(19) Europäisches Patentamt
European Patent Office
Office européen des brevets

(11) Numéro de publication: **0 311 467 B1**

(12) **FASCICULE DE BREVET EUROPEEN**

(45) Date de publication de fascicule du brevet: **04.08.93** (51) Int. Cl.[5]: **A61K 31/415**

(21) Numéro de dépôt: **88402156.9**

(22) Date de dépôt: **25.08.88**

# (54) Utilisation de dérivés de pyrrolo[3,2,1-hi]indole pour obtenir un médicament destiné au traitement du diabète.

(30) Priorité: **07.10.87 FR 8713831**

(43) Date de publication de la demande:
**12.04.89 Bulletin 89/15**

(45) Mention de la délivrance du brevet:
**04.08.93 Bulletin 93/31**

(84) Etats contractants désignés:
**AT BE CH DE ES FR GB GR IT LI LU NL SE**

(56) Documents cités:
**US-A- 4 617 313**

**ACTA DIABET. LAT., vol. 22, no. 3, 1985; M.D. WIDER et al., pp. 263-269&NUM;**

**DIABETES RESEARCH AND CLINICAL PRACTICE, vol. 1, 1985, Elsevier Science Publishers B.V. (Biomedical Division); M.R. PERKINS et al., pp. 9-20&NUM;**

(73) Titulaire: **SYNTHELABO**
**22, Avenue Galilée**
**F-92350 Le Plessis Robinson(FR)**

(72) Inventeur: **Bigg, Dennis**
**30, rue Gérard Machet**
**F-81100 Castres(FR)**
Inventeur: **Langer, Salomon**
**92, rue Jouffroy**
**F-75017 Paris(FR)**
Inventeur: **Morel, Claude**
**25, rue Gaubriel Péri Cresly**
**F-78470 Magny Les Hameaux(FR)**
Inventeur: **Sevrin, Mireille**
**73, rue Raymond Losserand**
**F-75014 Paris(FR)**

(74) Mandataire: **Ludwig, Jacques et al**
**SYNTHELABO, Service Brevets, 22, avenue Galilée, B.P. 72**
**F-92352 Le Plessis Robinson Cédex (FR)**

EP 0 311 467 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet l'utilisation de dérivés de pyrrolo[3,2,1-hi]indole pour obtenir un médicament destiné au traitement du diabète de type II.

Ces dérivés, décrits dans le brevet des Etats-Unis d'Amérique N° 4617313, répondant à la formule générale (I)

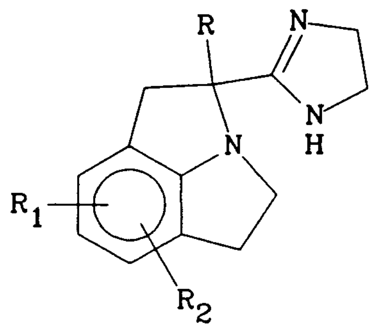

(I)

dans laquelle
R représente un atome d'hydrogène ou un radical ($C_{1-4}$)alkyle droit ou ramifié, et
$R_1$ et $R_2$ représentent chacun, indépendamment l'un de l'autre, un atome d'hydrogène, un atome d'halogène ou un radical ($C_{1-4}$)alkyle.
Les composés existent sous deux formes énantiomères ; ils peuvent donc se présenter sous forme d'énantiomères purs ou de mélanges d'énantiomères. Ils peuvent aussi exister à l'état de bases libres ou de sels d'addition à des acides.

Ils peuvent être préparés selon le procédé décrit dans le brevet cité ci-dessus. Les énantiomères peuvent être obtenus à partir des racémates, par cristallisation fractionnée des sels d'addition qu'ils forment avec les énantiomères d'acides chiraux, tels que l'acide tartrique et l'acide dibenzoyltartrique.

Selon le brevet cité ci-dessus, les composés de formule générale (I) sont des antagonistes des récepteurs adrénergiques $\alpha_2$ ; par ailleurs selon *Acta Diabet. Lat.*, **22**(3), 263-269 (1988) on sait que de tels antagonistes sont susceptibles de stimuler la sécrétion d'insuline chez les diabétiques de type II.

Les exemples suivants illustrent la préparation des composés utilisables selon l'invention. Les microanalyses et les spectres IR et RMN confirment les structures des produits obtenus.

### Exemple 1

(Dihydro-4,5 1H-imidazolyl-2)-2 propyl-2 tétrahydro-1,2,4,5 pyrrolo[3,2,1-hi]indole.

a) Tétrahydro-1,2,4,5 pyrrolo[3,2,1-hi]indolecarboxylate-2 d'éthyle.

Dans un ballon tricol de 1000 ml muni d'une agitation magnétique, d'une arrivée de chlorure d'hydrogène gazeux, d'un réfrigérant à air avec garde à chlorure de calcium, d'un thermomètre, et placé dans un bain de glace carbonique et d'alcool isopropylique, on introduit 15,8 g (0,073 mole) de dihydro-4,5 pyrrolo[3,2,1-hi]indolecarboxylate-2 d'éthyle et 150 ml d'éthanol.
On refroidit le mélange à -20 °C et on condense le gaz chlorhydrique à cette température jusqu'à obtenir une solution. On ajoute alors en une seule fois 26,1 g (0,22 atome-gramme) d'étain en grenaille, on retire le bain froid et on maintient l'agitation pendant 20 h à la température ambiante.
On obtient une suspension de couleur jaune, on la concentre au bain-marie et on la reprend avec 550 ml d'éthanol absolu. On refroidit le mélange, on y fait barboter de l'ammoniac jusqu'à pH = 9 à 10 pour précipiter les sels d'étain, on essore ces derniers, en les lavant à l'éthanol glacé, et on évapore à sec le filtrat obtenu. On soumet le résidu à une chromatographie sur colonne de silice en éluant avec du dichlorométhane. On recueille finalement 11,65 g d'un produit jaune huileux.

b) Propyl-2 tétrahydro-1,2,4,5 pyrrolo[3,2,1-hi]indolecarboxylate-2 d'éthyle.

Dans un ballon de Keller de 150 ml, muni d'une agitation magnétique, d'un thermomètre, d'une arrivée d'argon, d'une ampoule à introduction et placé dans un bain froid, on introduit sous atmosphère d'argon 3,4

ml (2,43 g ou 0,024 mole) de diisopropylamine et 20 ml de tétrahydrofuranne.
On refroidit le mélange réactionnel à -75 ° C et introduit 15 ml (0,024 mole) de butyllithium en solution 1,6 M dans l'hexane en 15 mn.
On maintient la solution incolore et limpide sous agitation à -70 ° C pendant 1 h, puis on ajoute en 15 mn à -70 ° C une solution de 4,3 g (0,02 mole) de tétrahydro-1,2,4,5 pyrrolo [3,2,1-hi]indolecarboxylate-2 d'éthyle dans 15 ml de tétrahydrofuranne. On maintient sous agitation à -70 ° C pendant 1 h la solution de couleur jaune orange, et on ajoute en 15 mn à -70 ° C une solution de 9,8 ml (17 g ou 0,1 mole) d'iodure de n-propyle dans 10 ml de tétrahydrofuranne. On maintient sous agitation à -70 ° C pendant 1 h la suspension de couleur rougeâtre et on la laisse à température ambiante pendant 3 h. Puis on verse le mélange dans 400 ml d'eau glacée, on l'extrait à l'éther diéthylique en présence d'une solution de chlorure de sodium. On lave à l'eau et sèche sur sulfate de sodium, on sépare la phase organique et on l'évapore à sec au bain-marie sous vide.
On obtient 5,4 g d'un liquide de couleur brune.
On le purifie par chromatographie sur colonne de silice en éluant avec un mélange 99/1 de dichlorométhane/méthanol. Après traitement du mélange il reste 2,3 g de produit qu'on utilise tel quel dans l'étape suivante.

c) (Dihydro-4,5 1H-imidazolyl-2)-2 propyl-2 tétrahydro-1,2,4,5 pyrrolo[3,2,1-hi]indole (composé utilisable selon l'invention).

Dans un ballon de Keller de 50 ml muni d'une agitation magnétique, d'un réfrigérant à reflux, d'un thermomètre, d'une arrivée d'argon, d'une ampoule à introduction et d'un appareil de Dean-Stark, on introduit sous atmosphère d'argon 6 ml de toluène et 2,9 ml (0,5 g ou 0,0064 mole) de triméthylaluminium (à 25,2 % dans l'hexane).
On refroidit à -10 ° C et on ajoute en 10 mn une solution de 0,45 ml (0,4 g ou 0,0064 mole) d'éthylènediamine en solution dans 3 ml de toluène.
On maintient l'agitation pendant 10 mn à -10 ° C, on laisse revenir à 0 ° C puis on chauffe à 50 ° C et on ajoute à cette température en 10 mn une solution de 1,1 g (0,0042 mole) de n-propyl-2 tétrahydro-1,2,4,5 pyrrolo [3,2,1-hi]indolecarboxylate-2 d'éthyle en solution dans 6 ml de toluène.
On chauffe au reflux pendant 9 h et abandonne au repos sous argon à la température ambiante pendant la nuit.
Après avoir refroidi le mélange entier à -15 ° C, on l'hydrolyse avec 2,9 ml d'eau tout en agitant, puis on l'extrait avec de l'acétate d'éthyle. On réunit les fractions organiques, on les lave avec une solution de chlorure de sodium, on les sèche, on les filtre et on les évapore.
On obtient 1 g d'un résidu de couleur marron clair dont on prépare directement le chlorhydrate.

d) Chlorhydrate de (dihydro-4,5 1H-imidazolyl-2)-2 propyl-2 tétrahydro-1,2,4,5 pyrrolo[3,2,1-hi]indole (composé utilisable selon l'invention).

Dans un ballon de 1000 ml muni d'un système d'agitation magnétique et placé sous atmosphère d'argon on introduit 5,1 g (0,02 mole) de (dihydro-4,5 1H-imidazolyl-2)-2 propyl-2 tétrahydro-1,2,4,5 pyrrolo-[3,2,1-hi]indole, 200 ml d'éther et 100 ml d'éther chlorhydrique 0,1 N. Le sel se forme instantanément et on filtre rapidement.
On reprend dans l'éther et on évapore sous vide. On met ensuite le sel en suspension dans l'acétone, on agite à 20 ° C pendant 30 mn. On filtre et on sèche sous vide. On recristallise le résidu dans l'alcool isopropylique.
F = 255 ° C (décomposition).

e) Enantiomère dextrogyre (composé utilisable selon l'invention).

On traite 25 g (0,1 mole) de dl (dihydro-4,5 1H-imidazolyl-2)-2 propyl-2 tétrahydro-1,2,4,5 pyrrolo[3,2,1-hi]indole en solution dans 200 ml de méthanol par une solution de 15,05 g (0,1 mole) d'acide (+)tartrique dans 200 ml de méthanol. On agite la solution à 20 ° C pendant 15 mn, on évapore le solvant sous vide et on fait recristalliser le résidu 3 fois dans le méthanol.
On obtient 8,2 g de sel.

$$F = 225\text{-}228°C \ [\alpha]_D^{20} = +134°C \ (c = 0,20 \ ; \ MeOH)$$

A une suspension de 6,1 g (0,015 mole) de ce sel dans 200 ml d'acétate d'éthyle, on ajoute 20 ml d'ammoniaque, on agite 5 mn à 20 °C, on décante, on extrait la phase aqueuse à l'acétate d'éthyle, on lave la phase organique à l'eau et on la sèche sur sulfate de magnésium. On obtient 3,8 g de base.

$$[\alpha]_D^{20} = +9,7° \ (c = 0,32 \ ; \ MeOH).$$

A une solution de 3,5 g (0,014 mole) de base dans 50 ml d'alcool isopropylique, on ajoute 140 ml d'une solution 0,1 N d'acide chlorhydrique dans l'alcool isopropylique. On agite la solution pendant 5 mn à 20 °C puis on évapore l'alcool sous vide. On lave le sel à l'acétate d'éthyle.

$$F = 249\text{-}251°C \ [\alpha]_D^{20} = +173° \ (c = 0,224 \ ; \ EtOH).$$

f) Enantiomère lévogyre

On met le résidu (21 g) provenant de l'évaporation des eauxmères de cristallisation du tartrate de l'isomère dextrogyre en suspension dans 300 ml d'acétate d'éthyle, puis on le traite par 100 ml d'ammoniaque. On agite 5 mn puis on décante, on extrait la phase aqueuse à l'acétate d'éthyle, on lave la phase organique à l'eau puis on la sèche sur sulfate de magnésium. On obtient 13 g de base.
On traite 7,25 g (0,028 mole) de base en solution dans 200 ml de méthanol par une solution de 4,3 g (0,028 mole) d'acide (-)tartrique dans 100 ml de méthanol.
Après 5 mn à 20 °C, on évapore le solvant sous vide et on recristallise le résidu dans le méthanol.

$$F = 227\text{-}230°C \ [\alpha]_D^{20} = -133° \ (C = 0,23 \ ; \ MeOH).$$

A une suspension de 6,2 g (0,0153 mole) de ce sel dans 200 ml d'acétate d'éthyle on ajoute 20 ml d'ammoniaque, on agite 5 mn à 20 °C, on décante, on extrait la phase aqueuse à l'acétate d'éthyle. On lave la phase organique à l'eau et on la sèche sur sulfate de magnésium.
On obtient 3,65 g de base.

$$[\alpha]_D^{20} = -9,5° \ (c = 0,22 \ ; \ MeOH).$$

A une solution de 3,5 g (0,014 mole) de la base dans 50 ml d'alcool isopropylique on ajoute 140 ml d'une solution 0,1 N d'acide chlorhydrique dans l'alcool isopropylique . On agite la solution 15 mn à 20 °C puis on évapore l'alcool sous vide et on lave le sel à l'acétate d'éthyle.

$$F = 249\text{-}251°C \ [\alpha]_D^{20} = -168,1° \ (c = 0,22 \ ; \ EtOH)$$

Exemple 2

(Dihydro-4,5 1*H*-imidazolyl-2)-2 fluoro-8 tétrahydro-1,2,4,5 pyrrolo[3,2,1-*hi*]indole.

a) N,N-diméthyl (fluoro-4 nitro-2 phényl)-2 éthènamine.

Dans un ballon de 1000 ml muni d'un agitateur magnétique, d'un réfrigérant et placé sous argon on introduit 60,6 g (0,39 mole) de fluoro-4 nitro-2 toluène, 143 g (1,2 mole) de (diméthoxyméthyl)-diméthylméthylamine et 400 ml de diméthylformamide.

On chauffe le mélange au reflux pendant 6 h, puis on ajoute encore 47,7 g (0,4 mole) de N,N-diméthyl diméthoxyméthylamine en solution dans 150 ml de diméthylformamide.
Après 8 h d'agitation à reflux on laisse refroidir le mélange puis on le verse dans de l'eau glacée. On extrait

la phase aqueuse à l'éther, on lave la phase organique à l'eau, on la sèche sur sulfate de sodium, et on évapore le solvant. Il reste 82,8 g d'huile rougeâtre qu'on utilise telle quelle dans l'étape suivante.

b) Fluoro-6 1H-indole.

Dans un flacon de Parr de 1,7 l en acier inoxydable on introduit 41,4 g (0,197 mole) de l'huile obtenue selon 2a), 500 ml d'éthanol et 5 g de Nickel de Raney humide, et on effectue une hydrogénation sous environ 0,44 MPa de pression maximale pendant 30 mn.
Ensuite on filtre le catalyseur en le rinçant à l'éthanol, et on évapore le filtrat sous vide. On purifie le résidu par chromatographie sur colonne de silice en éluant avec du dichlorométhane.
On obtient 39,4 g de fluoro-6 1H-indole pur.
F : 68-70 °C.

c) Fluoro-6 dihydro-2,3 1H-indole.

On additionne par portions 91 g (1,45 mole) de cyanoborohydrure de sodium à une solution de 55,8 g (0,41 mole) de fluoro-6 1H-indole dans 1,2 l d'acide acétique, en maintenant la température du mélange réactionnel entre 10 et 15 °C. Puis on agite le mélange à 20 °C pendant 2 h 30, on le verse dans 6 kg de glace, et on ajoute de la soude jusqu'à pH>10.
On agite le mélange pendant 30 mn puis on l'extrait avec 3 l d'éther. Après lavage à l'eau et séchage de la phase organique sur sulfate de sodium, on l'évapore sous vide. Il reste 54,6 g de résidu qu'on utilise tel quel dans l'étape suivante

d) Fluoro-6 nitroso-1 dihydro-2,3 1H-indole.

Dans un ballon tricol de 1000 ml, muni d'un agitateur magnétique et d'une ampoule à introduction, on introduit les 54,6 g (0,4 mole) du composé obtenu selon 2c) et 400 ml de solution aqueuse d'acide sulfurique à 25 %.
On refroidit le mélange et, tout en maintenant la température entre -10 et -5 °C, on ajoute goutte à goutte une solution de 31,1 g (0,45 mole) de nitrite de sodium dans 60 ml d'eau. On agite encore la solution, devenue visqueuse, à -5 °C pendant 30 mn puis on l'extrait avec du dichlorométhane. On lave la phase organique à l'eau jusqu'à neutralité, on la sèche sur sulfate de sodium et on l'évapore sous vide.
Il reste 66,4 g de produit qu'on utilise tel quel dans l'étape suivante.

e) Fluoro-6 dihydro-2,3 1H-indolamine-1.

Dans un ballon tricol de 6 l muni d'un agitateur mécanique, d'un réfrigérant, d'un thermomètre et d'une ampoule à introduction et placé sous argon, on introduit 16,3 g (0,43 mole) d'hydrure d'aluminium et lithium et 500 ml de tétrahydrofuranne.
On chauffe la suspension à environ 50 °C et on ajoute goutte à goutte une solution de 64,6 g (0,39 mole) de fluoro-6 nitroso-1 dihydro-2,3 1H-indole dans 500 ml de tétrahydrofuranne. Ensuite on agite le mélange pendant 4 h, puis on l'hydrolyse en ajoutant successivement 14 ml d'eau, 14 ml de soude 1N et 42 ml d'eau.
On agite encore pendant 30 mn, puis on sépare le solide minéral par filtration, en le rinçant à l'éther, on sèche le filtrat sur sulfate de sodium et on chasse les solvants sous vide.
On obtient 37,6 g d'une huile qu'on utilise telle quelle dans l'étape suivante.

f) [Fluoro-6 dihydro-2,3 1H-indolyl)-1]imino-2 propanoate d'éthyle.

Dans un ballon de 1000 ml muni d'un agitateur magnétique et d'un réfrigérant, placé sous argon, on introduit 37,6 g (0,25 mole) de l'indolamine obtenue selon 2e), 300 ml d'éthanol, 32,5 g (0,28 mole) d'oxo-2 propanoate d'éthyle et 2 ml d'acide acétique, et on agite le mélange sous reflux pendant 2 h.
On laisse refroidir, on chasse les solvants sous vide et on purifie le résidu par chromatographie sur colonne de silice en éluant avec du dichlorométhane. On obtient 46,6 g de composé purifié.

g) Fluoro-8 dihydro-4,5 1H-pyrrolo[3,2,1-hi]indolecarboxylate-2 d'éthyle.

Dans un ballon de Keller de 250 ml muni d'un agitateur et placé sous argon, on introduit 37,1 g (0,15 mole) du composé obtenu selon 2f) et 105 ml d'acide acétique.
On chauffe à 80°C et on ajoute goutte à goutte, en 10 mn, 26 ml, soit 30 g (0,21 mole) d'ethérate de trifluorure de bore.
On laisse monter la température et on poursuit l'agitation à 90°C pendant 1 h.
Ensuite on verse le mélange dans 400 ml d'eau, on extrait la phase aqueuse à l'éther, on lave la phase éthérée avec une solution aqueuse saturée de bicarbonate de sodium, puis avec de l'eau, et on la sèche sur sulfate de sodium.
Après évaporation des solvants on purifie le résidu par chromatographie sur colonne de silice en éluant avec du dichlorométhane. On obtient 8,4 g de composé pur.
F = 98-100°C.

h) Fluoro-8 tétrahydro-1,2,4,5 pyrrolo[3,2,1-hi]indolecarboxylate-2 d'éthyle.

Dans un ballon tricol de 1000 ml muni d'un agitateur magnétique, d'un réfrigérant et d'un thermomètre, on introduit 170 ml d'éthanol qu'on sature par barbotage d'acide chlorhydrique.
On ajoute 14 g (0,06 mole) du composé obtenu selon 2g), on fait encore barboter de l'acide chlorhydrique à -40°C, puis on ajoute 30 g (0,25 at.g) d'étain en grenaille.
Ensuite on agite le mélange à 20°C pendant 24 h, on évapore le solvant sous vide, on ajoute 500 ml d'éthanol absolu, on refroidit le mélange à -10°C et on y fait passer un courant d'ammoniac jusqu'à pH>9.
On évapore l'alcool sous vide, on reprend le résidu avec 500 ml d'éther, on sépare le solide par filtration en le rinçant à l'éther, et on évapore le filtrat sous vide.
On obtient 13,6 g de composé qu'on utilise tel quel dans l'étape suivante.

i) (Dihydro-4,5 1H-imidazolyl-2)-2 fluoro-8 tétrahydro1,2,4,5 pyrrolo[3,2,1-hi]indole (composé utilisable selon l'invention).

Dans un ballon de Keller de 250 ml muni d'un agitateur magnétique, d'un thermomètre, d'un appareil de Dean-Stark, et placé sous argon, on introduit 90 ml de toluène, 58,4 ml, soit 10,1 g (0,14 mole) de triméthylaluminium à 25,2 % dans l'hexane, on refroidit le mélange à -10°C et, en 15 mn, on ajoute une solution de 9,4 ml, soit 8,4 g (0,14 mole) d'éthylènediamine dans 30 ml de toluène.
On laisse monter la température à 0°C en 10 mn, puis on chauffe le mélange à 50°C et, à cette température, on ajoute au mélange 13,3 g (0,0565 mole) du composé obtenu selon 2h), dissous dans 90 ml de toluène.
On agite le mélange à 110°C pendant 3 h, en éliminant l'hexane au moyen de l'appareil de Dean-Stark, puis on le refroidit à -10°C et on l'hydrolyse avec 59 ml d'eau.
On ajoute 200 ml d'acétate d'éthyle, on sépare le solide par filtration en le rinçant à l'acétate d'éthyle, on lave la phase organique avec une solution saturée de chlorure de sodium, on la sèche sur sulfate de sodium, et on chasse le solvant sous vide.
On recueille 13,4 g de composé brut.
F = 100-102°C.
Pour préparer le chlorhydrate on en introduit 13,4 g (0,0565 mole) dans un ballon de 1000 ml muni d'un agitateur, et 565 ml d'alcool isopropylique chlorhydrique à 0,1 mole/l. On agite la solution pendant 15 mn, on évapore le solvant sous vide et on fait recristalliser le résidu dans 80 ml d'éthanol. Après filtration et séchage à 90°C sous vide on obtient 7,3 g de cristaux.
Point de fusion : 261-263°C.

j) Enantiomère dextrogyre (composé utilisable selon l'invention).

On traite 8 g (0,035 mole) du racémate brut (base) obtenu selon 2i), en solution dans 250 ml de méthanol, avec 13,02 g (0,035 mole) d'acide D(+)dibenzoyltartrique. Après 1 h d'agitation à 20°C on évapore le solvant sous vide et on recristallise le résidu trois fois dans le méthanol, en conservant à chaque fois la liqueur mère. On isole ainsi 6 g de dibenzoyltartrate.
Point de fusion : 190-195°C.

$$[\alpha]_D^{22} = + 171°(c=0,2, MeOH).$$

On ajoute 200 ml d'acétate d'éthyle et 20 ml d'ammoniaque à ce sel, on agite le mélange pendant 5 mn, on sépare la phase organique, et on extrait encore une fois la phase aqueuse avec de l'acétate d'éthyle.
On réunit les phases organiques et, après lavage à l'eau, séchage sur sulfate de magnésium et évaporation, on obtient 2 g d'énantiomère dextrogyre sous forme de base libre.

$$F = 120\text{-}125°C.\ [\alpha]_D^{22} = + 173,5° \ (c = 0,275 ; MeOH).$$

On en prépare le chlorhydrate en dissolvant 1,8 g de base dans 20 ml d'alcool isopropylique et en ajoutant 85 ml d'une solution 0,1 N d'acide chlorhydrique dans l'alcool isopropylique. On agite la solution pendant 5 mn à 20 ° C, on évapore le solvant sous vide et on lave le résidu avec de l'acétate d'éthyle.

$$F = 262\text{-}265°C\ [\alpha]_D^{22} = + 225° \ (c=0,275 ; EtOH).$$

k) Enantiomère lévogyre.

On réunit et évapore les liqueurs mères des recristallisations effectuées selon 2j), et on met en suspension les 12 g de résidu dans 300 ml d'acétate d'éthyle. On ajoute 20 ml d'ammoniaque à ce sel, on agite le mélange pendant 5 mn, on sépare la phase organique, et on extrait encore une fois la phase aqueuse avec de l'acétate d'éthyle.
On réunit les phases organiques et, après lavage à l'eau, séchage sur sulfate de magnésium et évaporation, on obtient 4,7 g de base riche en énantiomère lévogyre.
On dissout cette dernière dans 200 ml de méthanol et on ajoute 7,5 g d'acide L(-)dibenzoyltartrique en solution dans 50 ml de méthanol. Après 5 mn d'agitation à 20 ° C on évapore le solvant sous vide et on recristallise le résidu deux fois dans le méthanol. On obtient 5,5 g de sel.

$$F = 187\text{-}190°C\ [\alpha]_D^{22} = -179,6° \ (c=0,3 ; MeOH).$$

A une suspension de 5,5 g du sel précédemment obtenu dans 200 ml d'acétate d'éthyle on ajoute 20 ml d'ammoniaque, on agite le mélange pendant 5 mn, on sépare la phase organique, et on extrait encore une fois la phase aqueuse avec de l'acétate d'éthyle.
On réunit les phases organiques et, après lavage à l'eau, séchage sur sulfate de magnésium et évaporation, on obtient 1,5 g d'énantiomère lévogyre sous forme de base libre.

$$F = 123\text{-}125°C\ [\alpha]_D^{22} = -171° \ (c=0,23 ; MeOH).$$

On en prépare le chlorhydrate en dissolvant 1,7 g de base dans 30 ml d'alcool isopropylique et en ajoutant 80 ml d'une solution 0,1 N d'acide chlorhydrique dans l'alcool isopropylique. On agite la solution pendant 5 mn à 20 ° C, on évapore le solvant sous vide et on lave le résidu avec de l'acétate d'éthyle.

$$F = 258\text{-}261°C\ [\alpha]_D^{22} = -222,6° \ (c = 0,2 ; EtOH).$$

Le tableau ci-après illustre les structures et propriétés physiques de quelques composés utilisables selon l'invention.

Tableau

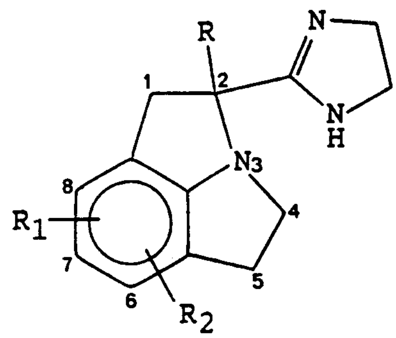

(I)

| Composé | $R_1$ | $R_2$ | R | Sel | F(°C) |
|---|---|---|---|---|---|
| 1 | H | H | $nC_3H_7$ | fumarate | 202-204 |
| isomère dextrogyre | | | | chlorhydrate<br>$[\alpha]_D^{20}=+173°$ | 249-251<br>(c=0,224 ; EtOH) |
| 2 | 8-F | H | H | fumarate | 174-176 |
| | | | | chlorhydrate | 261-263 |
| isomère dextrogyre | | | | chlorhydrate<br>$[\alpha]_D^{22}=+225°$ | 262-265<br>(c=0,275 ; EtOH) |

Les composés utilisables selon l'invention ont été étudiés chez la souris quant à leur effet sur la glycémie induite par administration orale de glucose.

On utilise des souris mâles CD d'un poids de 25 à 30 g, ayant librement accès à leur nourriture pendant tout l'essai. On leur administre, par voie i.p., une solution saline ou les composés utilisables selon l'invention, à raison de 10 mg/kg puis, 20 mn plus tard, on les traite par voie orale avec 1 g/kg de glucose en solution aqueuse. 30 mn plus tard on les décapite rapidement, on recueille le sang (0,5 à 1 ml) dans des tubes de centrifugation contenant un mélange d'héparine (50 u.i.), du fluorure de sodium et d'EDTA (Boehringer-Mannheim). On centrifuge immédiatement le sang et on recueille le plasma (200 à 700 µl) pour en doser le glucose.

Par rapport aux animaux de contrôle, qui ont reçu une solution saline, les animaux traités par les composés utilisables selon l'invention, en particulier par les composé N°1 et 2 (racémates) ou leurs énantiomères dextrogyres, présentent un taux de glucose significativement diminué, un effet dû très probablement à l'augmentation de la libération d'insuline par le pancréas.

Les résultats des essais montrent que les composés de l'invention sont utilisables pour la préparation de médicaments destinés au traitement des diabètes non insulino-dépendants (type II).

A cet effet ils peuvent être présentés sous toute forme convenant à l'administration orale ou parentérale, par exemple sous forme de comprimés, dragées, gélules, capsules, solutions ou suspensions buvables ou injectables, en association avec des excipients appropriés.

Ils peuvent être utilisés seuls ou combinés à d'autres substances actives telles que des antidiabétiques connus, ou à des substances destinées à renforcer leurs effets ou à prévenir ou atténuer les complications associées au diabète. Leur posologie peut aller de 5 à 100 mg de substance active par jour, par la voie orale.

## Revendications

**1.** Utilisation d'un composé de formule générale (I)

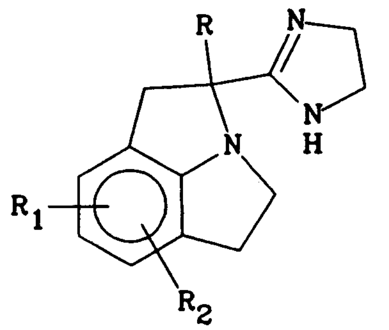

(I)

dans laquelle
soit R représente un atome d'hydrogène et $R_1$ représente un atome de fluor en position 8,
soit R représente un groupe propyle et $R_1$ représente un atome d'hydrogène, et
$R_2$ représente un atome d'hydrogène,
ledit composé étant sous forme d'énantiomère pur ou de mélange d'énantiomères, à l'état de base libre ou de sel d'addition à un acide acceptable en pharmacologie,
pour obtenir un médicament destiné au traitement du diabète de type II.

**2.** Utilisation selon la revendication 1, caractérisée en ce que le composé de formule (I) est le (dihydro-4,5 1H-imidazolyl-2)-2 propyl-2 tétrahydro-1,2,4,5 pyrrolo[3,2,1-hi]indole.

**3.** Utilisation selon la revendication 1, caractérisée en ce que le composé de formule (I) est l'énantiomère dextrogyre du (dihydro-4,5 1H-imidazolyl-2)-2 propyl-2 tétrahydro-1,2,4,5 pyrrolo[3,2,1-hi]indole.

**4.** Utilisation selon la revendication 1, caractérisée en ce que le composé de formule (I) est le (dihydro-4,5 1H-imidazolyl-2)-2 fluoro-8 tétrahydro-1,2,4,5 pyrrolo[3,2,1-hi]indole.

**5.** Utilisation selon la revendication 1, caractérisée en ce que le composé de formule (I) est l'énantiomère dextrogyre du (dihydro-4,5 1H-imidazolyl-2)-2 fluoro-8 tétrahydro-1,2,4,5 pyrrolo[3,2,1-hi]indole.

## Claims

**1.** Use of a compound of general formula (I)

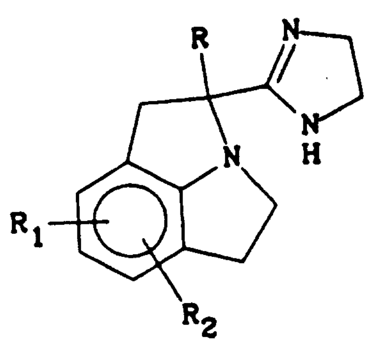

(I)

in which
either R represents a hydrogen atom and $R_1$ represents a fluorine atom at position 8,
or R represents a propyl group and $R_1$ represents a hydrogen atom, and
$R_2$ represents a hydrogen atom,

the said compound being in the form of a pure enantiomer or of a mixture of enantiomers, in the state of a free base or of an addition salt with a pharmacologically acceptable acid,
for obtaining a medicinal product intended for the treatment of type II diabetes.

**2.** Use according to Claim 1, characterised in that the compound of formula (I) is 2-(4,5-dihydro-1H-imidazol-2-yl)-2-propyl-1,2,4,5-tetrahydropyrrolo[3,2,1-hi]indole.

**3.** Use according to Claim 1, characterised in that the compound of formula (I) is the dextrorotatary enantiomer of 2-(4,5-dihydro-1H-imidazol-2-yl)-2-propyl-1,2,4,5-tetrahydropyrrolo[3,2,1-hi]indole.

**4.** Use according to Claim 1, characterised in that the compound of formula (I) is 2-(4,5-dihydro-1H-imidazol-2-yl)-8-fluoro-1,2,4,5-tetrahydropyrrolo[3,2,1-hi]indole.

**5.** Use according to Claim 1, characterised in that the compound of formula (I) is the dextrorotatary enantiomer of 2-(4,5-dihydro-1H-imidazol-2-yl)-8-fluoro-1,2,4,5-tetrahydropyrrolo[3,2,1-hi]indole.

**Patentansprüche**

**1.** Verwendung einer Verbindung der allgemeinen Formel (I)

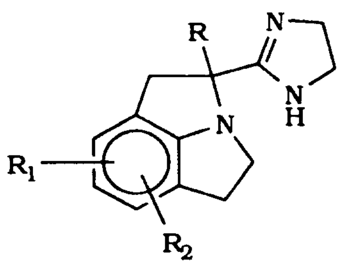

**(I)**

in der
entweder R ein Wasserstoffatom und $R_1$ ein Fluoratom in der 8-Stellung oder R eine Propylgruppe und $R_1$ ein Wasserstoffatom und
$R_2$ ein Wasserstoffatom bedeuten.
in Form des reinen Enantiomeren oder einer Mischung von Enantiomeren, in Form der freien Base oder eines Additionssalzes mit einer pharmakologisch annehmbaren Säure,
zur Herstellung eines Arzneimittels zur Behandlung von Diabetes Typ II.

**2.** Verwendung nach Anspruch 1, **dadurch gekennzeichnet,** daß die Verbindung der Formel (I) 2-(4,5-Dihydro-1H-imidazol-2-yl)-2-propyl-1,2,4,5-tetrahydro-pyrrolo[3.2.1-hi]indol ist.

**3.** Verwendung nach Anspruch 1, **dadurch gekennzeichnet,** daß die Verbindung der Formel (I) das rechtsdrehende Enantiomere von 2-[4,5-Dihydro-1H-imidazol-2-yl)-2-propyl-1,2,4,5-tetrahydro-pyrrolo-[3,2,1-hi]indol ist.

**4.** Verwendung nach Anspruch 1, **dadurch gekennzeichnet,** daß die Verbindung der Formel (I) 2-(4,5-Dihydro-1H-imidazol-2-yl)-8-fluor-1,2,4,5-tetrahydro-pyrrolo[3,2,1-hi]indol ist.

**5.** Verwendung nach Anspruch 1, **dadurch gekennzeichnet,** daß die Verbindung der Formel (I) das rechtsdrehende Enantiomere von 2-(4,5-Dihydro-1H-imidazol-2-yl)-8-fluor-1,2,4,5-tetrahydro-pyrrolol-[3,2,1-hi]indol ist.